# EUROPÄISCHE PATENTANMELDUNG

(11) **EP 3 680 231 A1**
(43) Veröffentlichungstag der Anmeldung: **15.07.2020**
(21) Anmeldenummer: 20156880.5
(22) Anmeldetag: 19.11.2009
(51) Int. Cl.: C07C 231/02, C07C 233/09, C07C 233/11, C08F 220/56

(54) **VERFAHREN ZUR HERSTELLUNG VON N-ISOPROPYL(METH)ACRYLAMID**

(30) Priorität: 15.12.2008 DE 102008054612
(62) Teilanmeldung aus: 09756729.1
(71) Anmelder: Evonik Operations GmbH, 45128 Essen (DE)
(72) Erfinder: Knebel, Joachim, 64665 Alsbach-Hähnlein (DE); Karnbrock, Wilhelm, 64625 Bensheim (DE); Kerscher, Volker, 64354 Reinheim (DE)
(74) Vertreter: Evonik Patent Association

(57) **Zusammenfassung**

Es wird ein neues Verfahren beschrieben, das es erlaubt, N-Isopropyl(meth)acrylamid auf einfache Weise in hoher Reinheit und Ausbeute zu gewinnen.

## Beschreibung

### Gebiet der Erfindung

Es wird ein Verfahren beschrieben, das es erlaubt, N-Isopropyl(meth)acrylamid auf einfache Weise in hoher Reinheit und Ausbeute zu gewinnen, indem man Methacrylsäureanhydrid mit Isopropylamin zur Reaktion bringt, optional in einem Lösungsmittel. Das Amid fällt während der Reaktion aus und kann durch Filtration isoliert werden. Ein weiterer Produktanteil kann durch Neutralisation der als Nebenprodukt anfallenden Methacrylsäure aus dem Filtrat ausgefällt werden und so zusätzlich gewonnen werden. Alternativ kann die Methacrylsäure destillativ von darin gelöstem Produkt getrennt werden.

Überraschenderweise bildet sich im Gegensatz zum Lehrbuchwissen bei der äquimolaren Umsetzung der Reaktanden nahezu kein N-Isopropylammoniummethacrylat.

### Stand der Technik

Die **Ritter-Reaktion** dient zur Herstellung von Amiden aus Nitrilen und Substraten, die Carbeniumionen bilden können (wie zum Beispiel tertiäre oder sekundäre Alkohole in Gegenwart von starken Mineralsäuren). So kann aus (Meth)acrylnitril und Isopropanol N-Isopropyl(meth)acrylamid hergestellt werden. Die DE 31 31 096 beschreibt dieses Verfahren. Zur Aufarbeitung muss die als Lösungsmittel fungierende Säure neutralisiert werden, was große Mengen Abfallsalz erzeugt. Obendrein enthält das Produkt Verunreinigungen, zum Beispiel Methacrylamid.

### Aufgabenstellung

### Durchführung der Erfindung

Man erhält das substituierte (Meth)acrylamid auf einfache Weise, indem man das Anhydrid, ggf. in einem Lösungsmittel vorlegt und das Amin, ggf. unter Kühlung, zudosiert.

Nach Ende der Zugabe ist in der Regel bereits ein Teil des entstandenen Produkts auskristallisiert und kann, ggf. nach Kühlen der Produktlösung zur Vervollständigung der Fällung, abfiltriert werden. Zur Erhöhung der Ausbeute kann die in der Mutterlauge enthaltene (Meth)acrylsäure neutralisiert werden, was zu einer weiteren Ausfällung von Produkt führt. Die Produktbildung erfolgt nahezu quantitativ. Das erhaltene substituierte (Meth)acrylamid besitzt eine hohe Reinheit, die gewöhnlich oberhalb von 95 % liegt. Zur Aufarbeitung kann die Mutterlauge auch destillativ in N-Isopropylmethacrylamid und Methacrylsäure getrennt werden. Wegen der Polymerisationsempfindlichkeit der Monomeren ist es zweckmäßig, dabei die thermische Belastung zu minimieren, z.B. durch Verwendung eines Dünnschichtverdampfers. Reinheit und Ausbeute der erhaltenen Monomere sind höher als bei Herstellung nach bekannten Verfahren wie der Ritter-Reaktion oder dem Einsatz von Carbonsäurechlorid als Rohstoff.

### Die Ausgangsstoffe

### Anhydride der ungesättigten Carbonsäuren

Als Anhydrid der ungesättigten Carbonsäuren kommen Acrylsäureanhydrid, Methacrylsäureanhydrid oder Itaconsäureanhydrid in Frage.

Das Methacrylsäureanhydrid wird z. B. von der Evonik Röhm GmbH in den Handel gebracht.

### Die Amine

Als Amine können primäre Amine und sekundäre Amine eingesetzt werden. Als primäre Amine kommen primäre ggf. substituierte aliphatische Amine in Frage, wie beispielsweise Methylamin, Ethylamin, Propylamin, Butylamin, Pentylamin, Hexylamin, Heptylamin, Octylamin, Dodecylamin, Isopropylamin, Isobutylamin und Benzylamin sowie Allylamin.

Ferner können primäre cycloaliphatische Amine eingesetzt werden, wie beispielsweise Cyclopropylamin, Cyclobutylamin, Cyclopentylamin und Cyclohexylamin.

Als primäre aromatische Amine können Anilin, die isomeren Aminotoluole, einzeln oder in Mischungen und die isomeren Xylidine, einzeln oder in Mischungen eingesetzt werden. Diese Verbindungen können gegebenenfalls durch ein oder mehrere Halogene substituiert sein.

Sekundäre aliphatische Amine, wie beispielsweise Dimethylamin, Methylethylamin, Diethylamin, Dipropylamin, Diisopropylamin, Dibutylamin, Dipentylamin, Dihexylamin, Diheptylamin und Dioctylamin können ebenfalls eingesetzt werden.

### Die Polymerisationsinhibitoren

Polymerisationsinhibitoren sind bereits bekannt. So können beispielsweise 1,4-Dihydroxybenzole zur Stabilisierung zugegeben werden. Es können jedoch auch anders substituierte Dihydroxybenzole zum Einsatz kommen. Allgemein lassen sich derartige Inhibitoren mit der allgemeinen Formel (I) wiedergeben 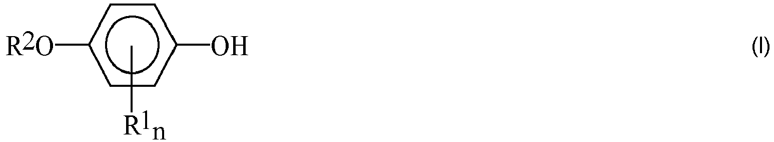 worin
R¹ Wasserstoff, einen linearen oder verzweigten Alkylrest mit eins bis acht Kohlenstoffatomen, Halogen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert.-Butyl, Cl, F oder Br;
n eine ganze Zahl im Bereich von eins bis vier, vorzugsweise eins oder zwei ist; und
R² Wasserstoff, einen linearen oder verzweigten Alkylrest mit eins bis acht Kohlenstoffatomen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl oder tert.-Butyl.

Es können jedoch auch Verbindungen mit 1,4-Benzochinon als Stammverbindung eingesetzt werden. Diese lassen sich mit der Formel (II) beschreiben 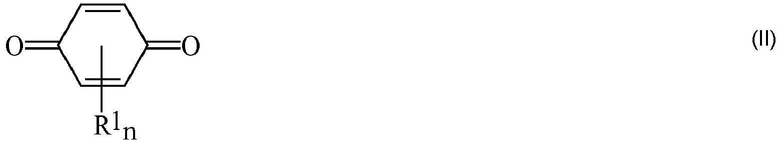 worin
R¹ einen linearen oder verzweigten Alkylrest mit eins bis acht Kohlenstoffatomen, Halogen oder Aryl bedeutet, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert.-Butyl, Cl, F oder Br; und

n eine ganze Zahl im Bereich von eins bis vier, vorzugsweise eins oder zwei ist.
Ebenso werden Phenole der allgemeinen Struktur (III) eingesetzt. 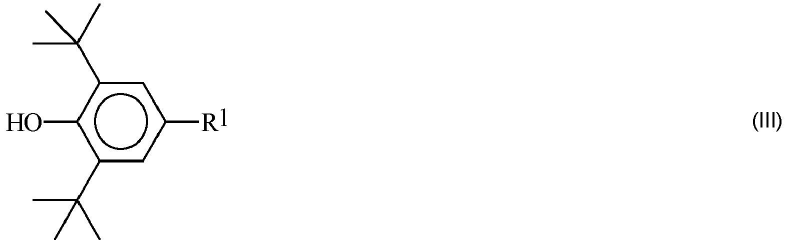 worin
R¹ einen linearen oder verzweigten Alkylrest mit eins bis acht Kohlenstoffatomen, Aryl oder Aralkyl, Proprionsäureester mit 1 bis 4 wertigen Alkoholen, welche auch Heteroatome wie S, O und N enthalten können, vorzugsweise einen Alkylrest mit eins bis vier Kohlenstoffatomen, besonders bevorzugt Methyl, Ethyl, n-Propyl, iso-Propyl, n-Butyl, iso-Butyl, sec-Butyl, tert.-Butyl, bedeutet.

Eine weitere vorteilhafte Substanzklasse stellen sterisch gehinderte Phenole auf Basis von Triazinderivaten der Formel (IV) dar: 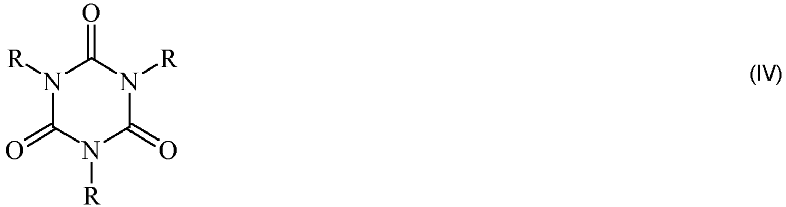 mit R = Verbindung der Formel (V) 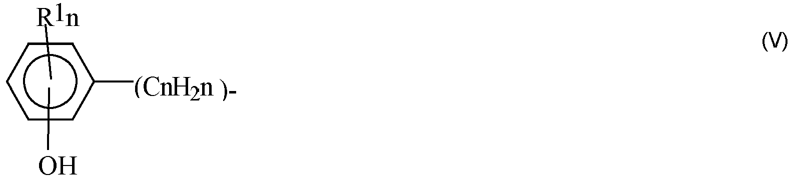 worin
R¹ = CₙH₂ₙ₊₁
mit n = 1 oder 2 ist.

Eine weitere Gruppe von bekannten Inhibitoren sind Amine, insbesondere sterisch gehinderte Amine.

Zu diesen gehören insbesondere Phenylendiamine, die durch Formel (VI) darstellbar sind 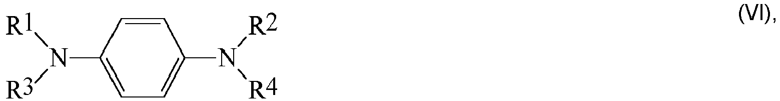 worin R¹, R², R³ und R⁴ unabhängig Wasserstoff sowie Alkyl-, Aryl-, Alkaryl-, Aralkyl-Reste mit jeweils bis zu 40, vorzugsweise bis zu 20 Kohlenstoffatomen darstellen, wobei vorzugsweise mindestens einer der Reste R¹, R², R³ und R⁴ Wasserstoff ist.
Beispielhafte p-Phenylendiamine umfassen p-Phenylendiamin worin die Reste R¹, R², R³ und R⁴ Wasserstoff sind; N-Phenyl-N'-alkyl-p-phenylendiamine wie beispielsweise, N-Phenyl-N'-methyl-p-phenylendiamin, N-Phenyl-N'-ethyl-p-phenylendiamin, N-Phenyl-N'-propyl-p-phenylendiamin, N-Phenyl-N'-isopropyl-p-phenylendiamin, N-Phenyl-N'-n-butyl-p-phenylendiamine, N-Phenyl-N'-isobutyl-p-phenylendiamin, N-Phenyl-N'-sec-butyl-p-phenylendiamin, N-Phenyl-N'-tert-butyl-p-phenylendiamin, N-Phenyl-N'-n-pentyl-p-phenylendiamin, N-Phenyl-N'-n-hexyl-p-phenylendiamin, N-Phenyl-N'-(1-methylhexyl)-p-phenylendiamin, N-Phenyl-N'-(1,3-dimethylbutyl)-p-phenylendiamin, N-Phenyl-N'-(1,4-dimethyl-pentyl)-p-phenylendiamin; N-Phenyl-N',N'-dialkyl-p-phenylendiamine, wie beispielsweise N-Phenyl-N',N'-dimethyl-p-phenylendiamin, N-Phenyl-N',N'-diethyl-p-phenylendiamin, N-Phenyl-N',N'-di-n-butyl-p-phenylendiamin N-Phenyl-N',N'-di-sec-butyl-p-phenylendiamin, N-Phenyl-N'-methyl-N'-ethyl-p-phenylendiamin; N,N-Dialkyl-p-phenylendiamine, wie beispielsweise N,N-Dimethyl-p-phenylendiamin und N,N'-Diethyl-p-phenylendiamin; N,N'-Dialkyl-p-phenylendiamine, wie beispielsweise N,N'-Diisopropyl-p-phenylendiamin, N,N'-Diisobutyl-p-phenylendiamin;
N,N'-Diaryl-phenylendiamine, wie beispielsweise N,N'-Diphenyl-p-phenylendiamin; N,N,N'-Trialkyl-p-phenylendiamine, wie beispielsweise N,N,N'-Trimethyl-p-phenylendiamin, N,N,N'-Triethyl-p-phenylendiamin.

Darüber hinaus bilden Phenazin-Farbstoffe eine weitere bevorzugte Gruppe. Diese umfassen insbesondere Indulin und Nigrosin. Nigrosin entsteht durch Erhitzen von Nitrobenzol, Anilin und salzsaurem Anilin mit metallischem Eisen und FeCl₃. Hierbei sind alkohollösliche Anilinfarbstoffe bevorzugt, die beispielsweise 5 Benzolkerne umfassen können, wie Dianilido-N,N-diphenylphenosafranin. Diese Stoffe sind weithin bekannt und können kommerziell erhalten werden.

Besonders erfolgreich werden die Verbindungen 1,4-Dihydroxybenzol, 4-Methoxyphenol, 2,5-Dichloro-3,6-dihydroxy-1,4-benzochinon, 1,3,5-Trimethyl-2,4,6-tris-(3,5-di-tert.butyl-4-hydroxybenzyl)benzol, 2,6-Di-tert. butyl-4-methylphenol, 2,4-Dimethyl-6-tert. butylphenol, 2,2-Bis [3,5-Bis(1,1-dimethylethyl)-4-hydroxyphenyl-1-oxopropoxymethyl)]1,3-propandiylester, 2,2'-Thiodiethylbis-[3-(3,5-di-tert.butyl-4-hydroxyphenyl)]propionat, Octadecyl-3-(3,5-di-tert.butyl-4-hydroxyphenyl)propionat, 3,5-Bis(1,1-dimethylethyl-2,2-Methylenbis-(4-methyl-6-tert.butyl)phenol, Tris-(4-tert.butyl-3-hydroxy-2,6-dimethylbenzyl)-s-triazin-2,4,6-(1H,3H,5H)trion, Tris (3,5-ditert.butyl-4-hydroxy)-s-triazin-2,4,6-(1H,3H,5H) trion, tert.-Butyl-3,5-dihydroxybenzol oder Diphenyl-p-phenylendiamin (DPPD) sowie 4-Hydroxy-2,2,6,6-tetramethylpiperidin-1-oxyl eingesetzt, wobei hiervon wiederum ganz besonders zweckmäßig Hydrochinonmonomethylether (4-Methoxyphenol) ist.

Die genannten Inhibitoren sind kommerziell erhältlich.

Als Grundstabilisierung für ethylenisch ungesättigte Verbindungen können die genannten Verbindungen allein oder in Mischung von zwei oder mehr Verbindungen eingesetzt werden. Sofern es sich um phenolische Verbindungen handelt, ist die Anwesenheit von Sauerstoff im Reaktionsgemisch erforderlich, um eine ausreichende Wirksamkeit gegen Polymerisation sicherzustellen. Dabei ist die Verwendung von Luft als Sauerstoffquelle besonders bevorzugt.

### Das Lösungsmittel

Als Lösungsmittel können alle inerten organischen Lösungsmittel verwendet werden, wie beispielsweise aliphatische Kohlenwasserstoffe, wie Hexan, Heptan, Oktan, Cyclohexan oder Gemische von aliphatischen Kohlenwasserstoffen, wie beispielsweise Petroläther, Ligroin, Dekalin, oder Benzin.

Ferner können aromatische Lösungsmittel, wie beispielsweise Benzol, Toluol oder die isomeren Xylole und Gemische aus vorstehend genannten Verbindungen, eingesetzt werden.

Ferner kommen sauerstoffhaltige Kohlenwasserstoffe in Frage, wie beispielsweise Diethylenglycolmonomethylether, Diethylenglycolmonoethylether, Triethylenglycolmonomethylether, Triethylenglycolmonoethylether, Diethylenglycolmonobutylether, Diethylether oder Methyl-tert.butylether. Auch Wasser kommt als inertes Lösungsmittel in Betracht, da es unter den Reaktionsbedingungen der Amidherstellung nur wenig Hydrolyse des (Meth)acrylsäureanhydrids bewirkt.

### Beispiele

### Beispiel 1 (nicht erfindungsgemäß): Herstellung von N-Isopropylmethacrylamid

In einem 2I-Fünfhalsrundkolben mit mechanischem Rührer, Tropftrichter, Lufteinleitrohr, Innentemperaturfühler und Rückflußkühler legt man 463 g (3 mol) Methacrylsäureanhydrid, 504 g (6 mol) Cyclohexan sowie als Inhibitor 0,077 g (120 ppm bez. auf Reaktanden) 2,6-Di(tert.butyl)-4-methylphenol vor und dosiert in 1,25 h bei einer Temperatur unter 20°C 177 g (3 mol) Isopropylamin unter Rühren und Einleiten eines langsamen Luftstroms zu. Dabei scheiden sich Kristalle ab. Nach Ende der Zugabe kühlt man die Suspension auf 4°C bis 8°C ab und rührt noch 2,5 h zur Vervollständigung der Fällung. Danach wird filtriert und die Kristalle werden zweimal mit je 60 g Cyclohexan gewaschen und an der Luft getrocknet. Ausbeute 188 g (49%) N-Isopropylmethacrylamid, Reinheit 97,8 % (durch Gaschromatographie ermittelt).

Die Mutterlauge wird zur Neutralisation der enthaltenen Methacrylsäure mit 280 g 30%iger Natronlauge versetzt, wobei sich zwei Phasen bilden. Man rührt 0,5 h bei Raumtemperatur, wobei kristalliner Feststoff ausfällt. Die Suspension wird zur Vervollständigung der Fällung 12 h bei 5°C gelagert. Danach wird filtriert und der Filterkuchen wird an der Luft getrocknet.

Ausbeute: 165 g (43% d. Th.) N-Isopropylmethacrylamid, Reinheit 93,8 % (durch Gaschromatographie ermittelt).

### Beispiel 2: Herstellung von N-Dodecylmethacrylamid

In einem 11-Fünfhalsrundkolben mit mechanischem Rührer, Tropftrichter, Lufteinleitrohr, Innentemperaturfühler und Rückflußkühler legt man 300 g (1,95 mol) Methacrylsäureanhydrid sowie als Inhibitor 0,06 g (120 ppm bez. auf Produkt) 2,6-Di(tert.butyl)-4-methylphenol sowie 0,03 g (60 ppm bez. auf Produkt) Hydrochinonmonomethylether sowie 0,003 g (6 ppm bez. auf Produkt) 4-Hydroxy-2,2,6,6-tetramethylpiperidin-1-oxyl vor und dosiert in 1,3 h bei einer Temperatur von 30...35°C 361 g (1,95 mol) aufgeschmolzenes (Fp. 25..28°C) N-Dodecylamin unter Rühren und Einleiten eines langsamen Luftstroms zu. Nach Ende der Zugabe rührt man den flüssigen leicht viskosen Ansatz 3 h bei 40..45°C nach und kühlt auf Raumtemperatur ab. Zum Abdestillieren der Methacrylsäure versieht man das Reaktionsgefäß mit einer Destillationsbrücke und erhitzt im Ölpumpenvakuum (1 mbar) innerhalb von 3h auf 97°C Sumpftemperatur, wobei 154 g Methacrylsäure (92 % d. Th.) erhalten werden. Der Destillationsrückstand besteht aus 490 g N-Dodecylmethacrylamid (99 % d. Th.) mit einer gaschromatographisch bestimmten Reinheit von 98%.

### Beispiel 3: Herstellung von N-Isopropylmethacrylamid

In einem 11-Vierhalsrundkolben mit mechanischem Rührer, Tropftrichter, Innentemperaturfühler und Rückflußkühler legt man 300 g (1,95 mol) Methacrylsäureanhydrid sowie als Inhibitor 0,06 g (120 ppm bez. auf Produkt) 2,6-Di(tert.butyl)-4-methylphenol sowie 0,03 g (60 ppm bez. auf Produkt) Hydrochinonmonomethylether sowie 0,003 g (6 ppm bez. auf Produkt) 4-Hydroxy-2,2,6,6-tetramethylpiperidin-1-oxyl vor und dosiert in 3 h bei einer Temperatur von max. 30°C 115 g (1,95 mol) Isopropylamin unter Rühren zu. Nach Ende der Zugabe rührt man den flüssigen leicht viskosen Ansatz 3 h bei 40°C nach und kühlt auf Raumtemperatur ab. Die ausgefallenen Kristalle werden abgesaugt und mit 157 g Cyclohexan gewaschen, das mit der Mutterlauge vereinigt wird. Man erhält als erste Fraktion 114 g (46% d. Th.) N-Isopropylmethacrylamid mit einem Schmelzpunkt von 89 °C und einer gaschromatographisch bestimmten Reinheit von 99 %. Zur Aufarbeitung der Mutterlauge führt man diese einem Labordünnschichtverdampfer DS 25 (Fa. NGW, Wertheim) mit aufgesetzter Vigreuxkolonne (Länge 10 cm) zu und zieht bei einer Heizmanteltemperatur von 130°C und einem Druck von 1 mbar das Cyclohexan und die Methacrylsäure ab. Der Destillationsrückstand erstarrt beim Abkühlen auf RT und besteht aus 130 g N-Isopropylmethacrylamid (52 % d. Th.) mit einer gaschromatographisch bestimmten Reinheit von 97 %.

### Vergleichsbeispiel 1: Herstellung von N-Isopropylmethacrylamid aus Methacrylsäurechlorid

In einem 11-Vierhalsrundkolben mit mechanischem Rührer, Tropftrichter, Innentemperaturfühler und Rückflußkühler legt man 500 g (1mol) 2n Natronlauge, 59 g (1 mol) Isopropylamin sowie als Inhibitor 0,1 g 2,4-Dimethyl-6-(tert.butyl)phenol vor und dosiert bei Raumtemperatur 104 g (1 mol) Methacrylsäurechlorid unter Rühren zu. Nach Ende der Zugabe rührt man den Ansatz 1 h nach, fügt nochmals 10 g Natriumhydroxid hinzu und rührt weitere 30 min. Die ausgefallenen Kristalle werden abgesaugt und an der Luft getrocknet. Man erhält 108 g (85 % d. Th.) N-Isopropylmethacrylamid mit einer gaschromatographisch bestimmten Reinheit von 90,7%. Als Nebenprodukt sind 9 % eines Additionsprodukts von Methacrylsäurechlorid an N-Isopropylmethacrylamid enthalten.

### Vergleichsbeispiel 2: Herstellung von N-Isopropylmethacrylamid aus Methacrylsäurechlorid

In einem 11-Vierhalsrundkolben mit mechanischem Rührer, Tropftrichter, Lufteinleitrohr, Innentemperaturfühler und Rückflußkühler legt man 300 ml Toluol sowie 118 g (2mol) Isopropylamin und als Inhibitor 0,1 g 2,6-Di(tert.butyl)-4-methylphenol vor und dosiert bei max. 30°C 104 g (1 mol) Methacrylsäurechlorid unter Rühren und Lufteinleitung zu. Das ausgefallene Produkt wird abgesaugt und getrocknet. Man erhält 65 g (51% d. Th.) N-Isopropylmethacrylamid mit einer gaschromatographisch bestimmten Reinheit von 82,4%. Als Nebenprodukt sind 1,3 % eines Additionsprodukts von Methacrylsäurechlorid an N-Isopropylmethacrylamid sowie 11 % Methacrylsäureanhydrid enthalten.

### Vergleichsbeispiel 3: Herstellung von N-Isopropylmethacrylamid aus Methacrylnitril nach Ritter in 100%iger Schwefelsäure

In einem 21-Vierhalsrundkolben mit mechanischem Rührer, Tropftrichter, Lufteinleitrohr, Innentemperaturfühler und Rückflußkühler legt man 515 g (5,25 mol) 100%ige Schwefelsäure vor und dosiert innerhalb von 2,5 h ein Gemisch von 168 g (2,5 mol) Methacrylnitril, 180 g (3 mol) Isopropanol und 0,18 g 2,6-Di(tert.butyl)-4-methylphenol unter Rühren und Lufteinleiten zu. Die exotherme Reaktion wird durch Kühlen im Bereich von 22 bis 25°C gehalten. Nach Ende der Zugabe lässt man 1h bei 30°C nachreagieren, erwärmt dann auf 60°C und hält das Reaktionsgemisch 1 h bei dieser Temperatur. Danach kühlt man auf Raumtemperatur und gibt innerhalb von 70 min. 750 g Wasser unter Kühlen hinzu, so dass die Temperatur nicht ansteigt. Man überführt den Ansatz in einen 41-Kolben und gibt weitere 925 g Wasser zu, wobei sich Feststoff abscheidet. Mit 50%iger Natriumhydroxidlösung (ca. 815 g) wird das Gemisch innerhalb von 120 min bei max. 30°C neutralisiert (pH=7). Man kühlt auf 18 °C, dann wird das ausgefallene Produkt abgesaugt und viermal mit je 320 ml kaltem Wasser gewaschen. Der erhaltene Kristallbrei wird abgepresst. Man erhält 1136 g Produkt, das neben N-Isopropylmethacrylamid 42 % Wasser und 22 % Natriumsulfat enthält. Der N-Isopropylmethacrylamidanteil weist eine gaschromatographisch bestimmte Reinheit von 93,5% auf und enthält 5,9 % Methacrylamid.

Die erfindungsgemäßen Verbindungen können als Monomere zur Herstellung von Polymerisaten und Copolymerisaten verwendet werden. Ferner können die erfindungsgemäßen Monomere zu Polymeren polymerisiert oder in Mischungen copolymerisiert werden, die als Gashydratbildungsinhibitoten verwendet werden.

### Bevorzugte Ausführungsformen

1. Verfahren zur Herstellung von Amiden ungesättigter Carbonsäuren,
   dadurch gekennzeichnet, dass
   man das Anhydrid der ungesättigten Carbonsäure mit einem Amin in einem inerten Lösungsmittel umsetzt und das entstehende Amid abtrennt.
2. Verfahren zur Herstellung von Amiden ungesättigter Carbonsäuren,
   dadurch gekennzeichnet, dass
   man das Anhydrid der ungesättigten Carbonsäure mit einem Amin lösungsmittelfrei umsetzt und das entstehende Amid abtrennt.
3. Verfahren zur Herstellung von Amiden ungesättigter Carbonsäuren gemäß Ausführungsform 1 oder 2,
   dadurch gekennzeichnet, dass
   man die Mutterlauge aus dem Amidabtrennschritt neutralisiert, um weiteres Amid auszufällen und das entstehende Amid abtrennt.
4. Verfahren zur Herstellung von Amiden ungesättigter Carbonsäuren gemäß Ausführungsform 1 oder 2,
   dadurch gekennzeichnet, dass
   man die Mutterlauge aus dem Amidabtrennschritt destillativ von (Meth)acrylsäure befreit und das zurückbleibende Amid abtrennt.
5. Verfahren zur Herstellung von Amiden ungesättigter Carbonsäuren,
   dadurch gekennzeichnet, dass
   man das Anhydrid der ungesättigten Carbonsäure mit einem primären Amin in einem inerten Lösungsmittel umsetzt und das entstehende Amid abtrennt.
6. Verfahren zur Herstellung von Amiden ungesättigter Carbonsäuren,
   dadurch gekennzeichnet, dass
   man das Anhydrid der ungesättigten Carbonsäure mit einem sekundären Amin in einem inerten Lösungsmittel umsetzt und das entstehende Amid abtrennt.
7. Verfahren zur Herstellung von Amiden ungesättigter Carbonsäuren,
   dadurch gekennzeichnet, dass
   man das Anhydrid der ungesättigten Carbonsäure mit einem primären Amin lösungsmittelfrei umsetzt und das entstehende Amid abtrennt.
8. Verfahren zur Herstellung von Amiden ungesättigter Carbonsäuren,
   dadurch gekennzeichnet, dass
   man das Anhydrid der ungesättigten Carbonsäure mit einem sekundären Amin lösungsmittelfrei umsetzt und das entstehende Amid abtrennt.
9. Verfahren zur Herstellung von N-Isopropylmethacrylamid,
   dadurch gekennzeichnet, dass
   man Methacrylsäureanhydrid mit Isopropylamin in einem Lösungsmittel umsetzt und das N-Isopropylmethacrylamid isoliert.
10. Verfahren zur Herstellung von N-Isopropylmethacrylamid,
   dadurch gekennzeichnet, dass
   man Methacrylsäureanhydrid mit Isopropylamin ohne Lösungsmittel umsetzt und das N-Isopropylmethacrylamid isoliert.
11. Verwendung der Monomere zur Herstellung von Polymeren oder Copolymeren.
12. Verwendung der Monomere zur Herstellung von Polymeren oder Copolymeren, die als Gashydratbildungsinhibitoren verwendet werden.
13. Polymer oder Copolymer, erhältlich durch Polymerisation des Monomers nach einer der Ausführungsformen 1 bis 8.

## Patentansprüche

1. Verfahren zur Herstellung von (Meth)acrylamiden aus (Meth)Acrylsäureanhydrid durch Umsetzung mit primären oder sekundären Aminen,
**dadurch gekennzeichnet, dass**
(a) das Amin zu einer Mischung enthaltend das Anhydrid und mindestens einen Stabilisator bei einer Temperatur von maximal 30 °C zudosiert wird,
(b) der Ansatz nach Abschluss der Zugabe bei einer Temperatur von 40 bis 45°C gehalten wird, und
(c) das entstandene Amid schließlich abgetrennt wird.

2. Verfahren nach Anspruch 1,
**dadurch gekennzeichnet, dass**
das (Meth)Acrylsäureanhydrid mit dem Amin lösungsmittelfrei umgesetzt wird.

3. Verfahren nach Anspruch 1 oder nach Anspruch 2,
**dadurch gekennzeichnet, dass**
das die Mutterlauge aus dem Amidabtrennschritt (c) destillativ von (Meth)acrylsäure befreit wird und das zurückbleibende Amid abtrennt wird.

4. Verfahren nach einem der Ansprüche 1 bis 3,
**dadurch gekennzeichnet, dass**
das es sich bei dem (Meth)acrylamid um N-Isopropylmethacrylamid, bei dem (Meth)Acrylsäureanhydrid um Methacrylsäureanhydrid und bei dem primären oder sekundären Amin um Isopropylamin handelt.
